# EUROPEAN PATENT APPLICATION

(11) **EP 1 746 105 A2**
(43) Date of publication of application: **24.01.2007**
(21) Application number: 06118756.3
(22) Date of filing: 26.05.2003
(51) Int. Cl.: C07K 14/705

(54) **Human 5-HT7 receptor promoter sequence**

(30) Priority: 31.05.2002 EP 02077309
(62) Divisional of application: 03732469.6
(71) Applicant: JANSSEN PHARMACEUTICA N.V., 2340 Beerse (BE)
(72) Inventor: Laenen, Koen Leander Maria, 9000 Gent (BE); Vanhoenacker, Peter Jerome Prospere, 9000 Gent (BE); Haegeman, Guy Christian Antoine Victor Emile, 9000 Gent (BE)

(57) **Abstract**

The present invention provides an isolated nucleic acid molecule constituting the human 5HT₇ receptor promoter. Also disclosed are fragments thereof including enhancers and/or responsive elements such as NcoI or BARBIE, as well as the recombinant use thereof. It is thus a further aspect of the present invention to provide recombinant DNA molecules wherein the nucleotides according to the invention are operably linked to a detectable product, such as a luciferase gene or a CAT gene. Cell-based screening assays for identifying compounds that modulate the level of expression of a gene operably linked to the human 5HT₇ receptor promoter, or an active fragment thereof, also are provided.

SEQUENCE LISTING

<110>  Janssen Pharmaceutica N.V.

<120>  Human 5-HT7 receptor promoter sequence

<130>  JAB 1720

<150>  EP 02077309.9

<151>  2002-05-31

<160>  6

<170>  PatentIn version 3.1

<210>  1

<211>  3081

<212>  DNA

<213>  Homo sapiens

<400>  1
tggtggctca tgcctgtaat ctcagcactt tgggaggctg aggcaggcgg atcacttgag
60

gttaagagtt tgaggccagc ctggccaaca tggtgaaacc cggtccctac taaaaacaat
120

acaaaaatta ggcaggtgtg gtggcatgtg cctgtaatcc cagctacttg ggaagctgag
180

gcagaagaat cgcttgaacc tgggaggttg aggttgcagt gagccgagat cgcgccactg
240

cactccagcc tgggtgacag agcaagaccc tgtctcaaaa aaaaaaaaaa aaaaaaaaaa
300

aaagaaagag agacccagag agctctccta ccccctttgc caggtgagga cacagcaaca
360

tggctaaatt tactgttgcc ttgttcttgg taaatgtttg ttgttaagcc acccaggcta
420

tgatattttt gttatggcat cccaaaggga ctaaaacaag acaaaagcaa aaactagaag
480

ctgttgtgaa atataaatgg gctacgtgag ggctgtaaga gtgtaggtgg taaatcatat
540

tgactagagg tttaaataca aataaaatga ttaattttca aaatctgtgt gtaattactc
600

tggaatatta tttgacagag ccaagacaga atatataagc tgaactgatt agggtattca
660

aggggaagaa tacaaaagtc aaaataagtt aaaatgagtt gaaaagttaa aatgaaaaaa
720

atatcaaaag tatcttgggg aatatactac atgaaggaat ttttaaaaga ccttaagaca
780

attccaaacc tcaaaataaa aaaatggaga catttcaatg cagtcacata tgatcttgga
840

taagttataa aatttctccc agcctctatt tcctcctctg cactgaaggg tgaacaaatg
900

gtttcttaga tgcattccag caacaaacta tttgtctttt gtctgagatt tcctctctcc
960

caagatcccc agtggatgcc taaaaccccg gatagtaccc aatcgtatat atactatgtt
1020

tttttttttg atctggtaag agagagggct actaagtgac taatggtgga tggcatatac
1080

agtgtggata tgttggacaa gggatgattc atatcctggg taagatagag atttcatcac
1140

gctactcaga atagtgtgca attgacaact tacacattgt ttatttctga aaatttccat
1200

ttaatatttt cggaccgtgg ttcaccacag ataacaaacc aagaaaagcg aaacagagaa
1260

taactaacat aagtaaggtt gaagaaattt cctcatccat cttaactgtt agataatatt
1320

gctttgtggc ctaggtatta tattcaactc ttcctctgac tcaatggatt ggtttgctta
1380

tcctaggcag agaggagcct ctgaaatgtt gatcacagca aaagagctgt gaacattagg
1440

gctttttttt ttttccatta aaactttttc tgaatgtgta cagggaagaa tatgggcata
1500

gtaatcagat aaacttgggt ttgaaacata actattcctt accagctgat tgcaagagct
1560

aacaaccttc cctatctgga aaaacagagc taatacccac cttgaagaat gaaagtacta
1620

ttcagcactc tgatcaatat actttgaggt tttattcctt tttcttctta tttataatcc
1680

gctagggcct aacaagaaaa aacagatttg attattttta gagtcttaaa ctacagaaat
1740

cactgctggc ttccaatgaa accttcaaac aaaatggaat gaagcaagtc tcacaaataa
1800

taaaaatcag ccgctctgac agggtttgga taccaagggg aattttagcc aataggtatt
1860

aaatcaacat acacctccag gattccttta tagaagagta taaccggtca tttctttgct
1920

tatgtgtaag acttccagaa tgctacggga aaattaactc tcttttcaat atattaggct
1980

ttgaagtcta aatataaagg caatcagggt ctattttcag agcggagagg actgtttcta
2040

gacatccagg aaggattagt ccaaatgggt gtaaccccct tggaggcctt gtccccagag
2100

catcctcatc tcctgggtgc caggggctta aggaatcccc ctccctcctt tttttctttc
2160

ttcctcttct cggagctccc accctcaccc cattcggctg cttcttttcc atcttcgctt
2220

cttcccataa gcggtcaaaa tagaaaacgg aagtacctat ttgttgctta gtgtagtgct
2280

cagagacgtt tttttggttg ttgtttaatt aagaaaaata aaatccccct ctaggttctt
2340

catttcgcaa tgagcccaaa catccgctgg ctcattaggc ctcttaacaa aagaaaaatg
2400

cccccactga actgtggctg actagtgtgc ggaggctggg gtcggctgga ggggttctaa
2460

gtatctatca tgcggcaagg acagcttccc gggcgagggg acccgggatc ccgggaggcg
2520

cgtgcagcgc cgccccctca cagtagggag gcgcgggaga ggcggcggtg cctgcccagc
2580

ccgggtatcc cgtcccagga gcgcgaggga gcgggcagcg gccggaggcg gcggcggcgg
2640

ggacgcggcg cggctgccgc aggggagcgg cggcggcggc ggcggcggcg gcgggcgcga
2700

ggggcggggc gcactccgca acttctgccg ctgccgcccg ggcgctctcg gcgcagccga
2760

gctgctgctg cgaactgtgg agctgccagc ggagcccagc ccgagctccc tcgactccgc
2820

gcgtcccagg cgtccgtcgg gggccgaagc gcctcccgca gggagtcccc gtgaggccgc
2880

gctgccgcgt tacccgcagc ggccgggggt ccggggagtg cggggctccg agacagagcc
2940

aggcgccccc cagcggtcgg cgcgggcccc atggctgggc cgggcggagc ggaaccggtg
3000

aggtgaagcc ccggggccgg cagccggagg cgcgtggccg gggcgccggc tccatgggca
3060

gcggcacacg gcggcgcgat g
3081

<210> 2

<211> 83

<212> DNA

<213> Homo sapiens

<400> 2
catggctggg ccgggcggag cggaaccggt gaggtgaagc cccggggccg gcagccggag
60

gcgcgtggcc ggggcgccgg ctc
83

<210>  3

<211>  15

<212>  DNA

<213>  Homo sapiens


<400>  3
ctccctcctt ttttt
15


<210>  4

<211>  30

<212>  DNA

<213>  Homo sapiens


<400>  4
gaggtgaagc cccggggccg gcagccggag
30


<210>  5

<211>  154

<212>  DNA

<213>  Homo sapiens


<400>  5
ccgagacaga gccaggcgcc ccccagcggt cggcgcgggc cccatggctg ggccgggcgg
60

agcggaaccg gtgaggtgaa gccccggggc cggcagccgg aggcgcgtgg ccggggcgcc
120

ggctccatgg gcagcggcac acggcggcgc gatg
154


<210>  6

<211>  233

<212>  DNA

<213>  Homo sapiens


<400>  6
gatcccggga ggcgcgtgca gcgccgcccc ctcacagtag ggaggcgcgg gagaggcggc
60

ggtgcctgcc cagcccgggt atcccgtccc aggagcgcga gggagcgggc agcggccgga
120

ggcggcggcg gcggggacgc ggcgcggctg ccgcagggga gcggcggcgg cggcggcggc
180

ggcggcgggc gcgaggggcg gggcgcactc cgcaacttct gccgctgccg ccc
233

## Description

The present invention relates to an isolated promoter region of the human 5-HT₇ receptor. The invention also relates to screening methods for agents modulating the expression of the human 5-HT₇ receptor and thereby being potentially useful for the treatment of medical conditions related to serotonin-mediated responses such as schizophrenia, depression, migraine, affective disorders and disregulation of sleep and gastrointestinal functions.

### BACKGROUND OF THE INVENTION

The 5-hydroxytryptamine (5-HT) system is probably the most diverse neurotransmitter system in the vertebrate CNS in terms of the receptor complement mediating its cellular actions. To date 14 serotonin (5-HT) receptors have been cloned and classified into seven 5-HT receptor subtypes. With the exception of the 5-HT₃ receptor, which is a ligand-gated ion channel, 5-HT receptors belong to the G-protein-coupled receptor (GPCR) superfamily. Among these, the 5-HT₇ receptor is the latest to be cloned, although its existence had long been suspected because of its unique pharmacology. In situ hybridization and ligand-binding approaches have mapped its distribution in the CNS and indicate highest abundance in the thalamus, hypothalamus and hippocampus with lower levels in the cerebral cortex and amygdala (Vanhoenacker et al., 2000).

Despite this insight into 5-HT₇ receptor localization, there have been relatively few reports regarding the physiological role of these receptors. Studies based on non-selective ligands suggest a role for the 5-HT₇ receptor in the regulation of circadian rhythm (Lovenberg et al., 1993; Ying and Rusak, 1997) as well as an association with sleep disorders and depression. Down-regulation of 5-HT₇-like binding sites after chronic antidepressant treatments further supports this role. RT/PCR studies have assisted in the identification of four mammalian 5-HT7 splice variants, that are structurally divergent in their predicted intracellular carboxyl termini (Vanhoenacker et al., 2000).

Serotonin receptor expression is often highly tissue-specific, and in the cases that have been studied, each serotonin receptor family member seems to have a characteristic pattern of regional expression within the brain. This restricted pattern of expression is probably a critical point of control for mediating specific biological responses to serotonin. So has it only recently been demonstrated that the 5-HT₇ receptor is expressed in the suprachiasmatic nucleus (SCN), a region which is implicated in circadian rhythm. Studies using 8-OH-DPAT, a 5-HT_{1A} agonist with high affinity for the 5-HT₇ receptor, also shifted the circadian rhythm of hamsters (Ehlen et al., 2001). These results suggest a potential role for the 5-HT₇ receptor in sleep regulation. In view of this restricted expression pattern, the 5-HT₇ receptor becomes an interesting canditate to study its gene regulation.

In this respect, it has already been shown that the 5-HT₇ receptor is downregulated by steroids in vivo (Le Corre et al., 1997; Yau et al. 1997) and in vitro (Shimizu et al., 1997). This down-regulation may play a role in affective disorders, and it is, accordingly, important to identify the regulatory sequence elements involved. Similarly, a down-regulation of 5-HT₇ receptors occurs after chronic antidepressant treatment (Sleight et al., 1995; Mullins et al., 1999). This and other preliminary results (Harsing et al., Soc. Neurosc Abstr. 380.1, 2001) suggests a presynaptic role for the 5-HT₇ receptor and therefore this receptor might also be involved in the negative feed back loop that controls serotonin release. In this respect, it would be beneficial to improve the selectivity of 5-HT₇ receptor antagonists which are currently available. The elucidation of 5-HT₇ promoter regulation will allow to interfere with 5-HT₇ receptor formation and lead to new screening strategies for drugs interfering with 5-HT₇ receptor function.

### SUMMARY OF THE INVENTION

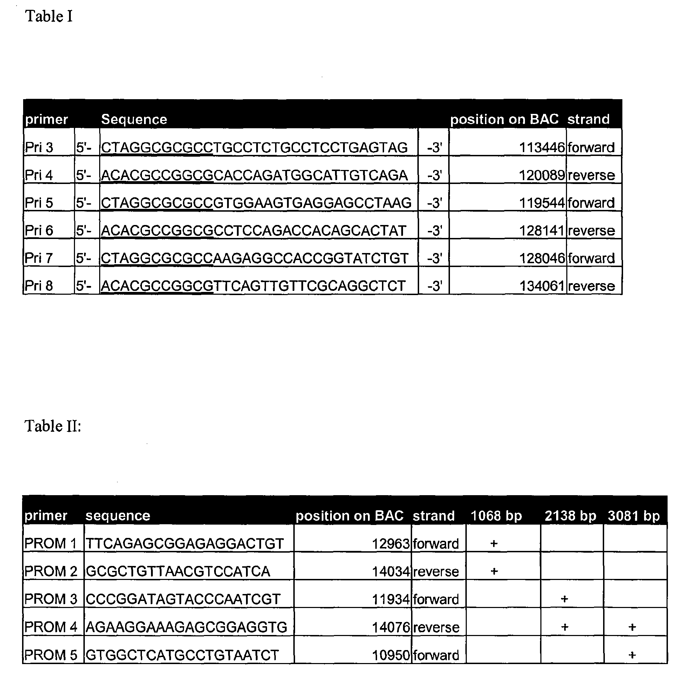

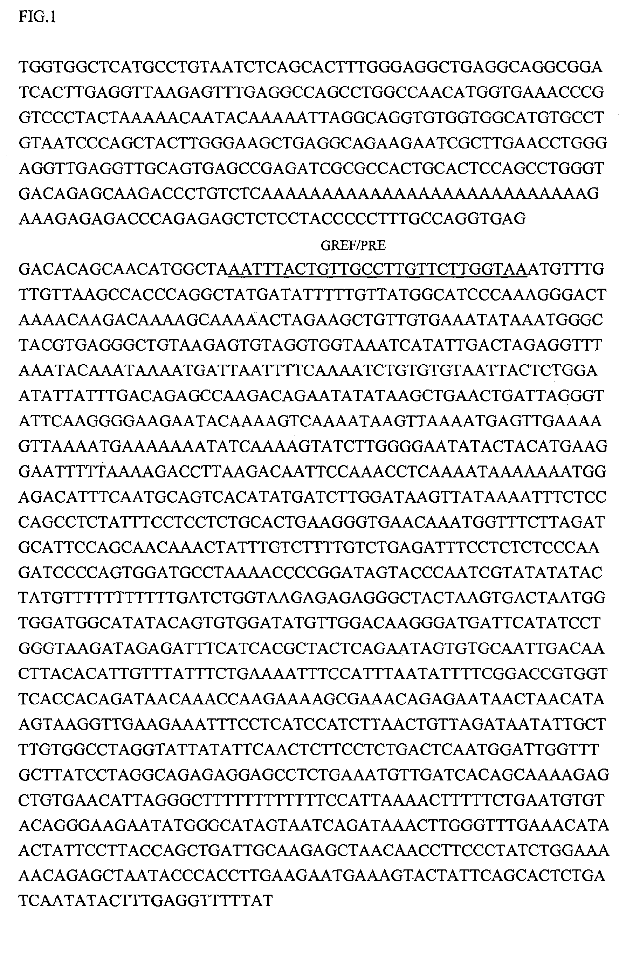

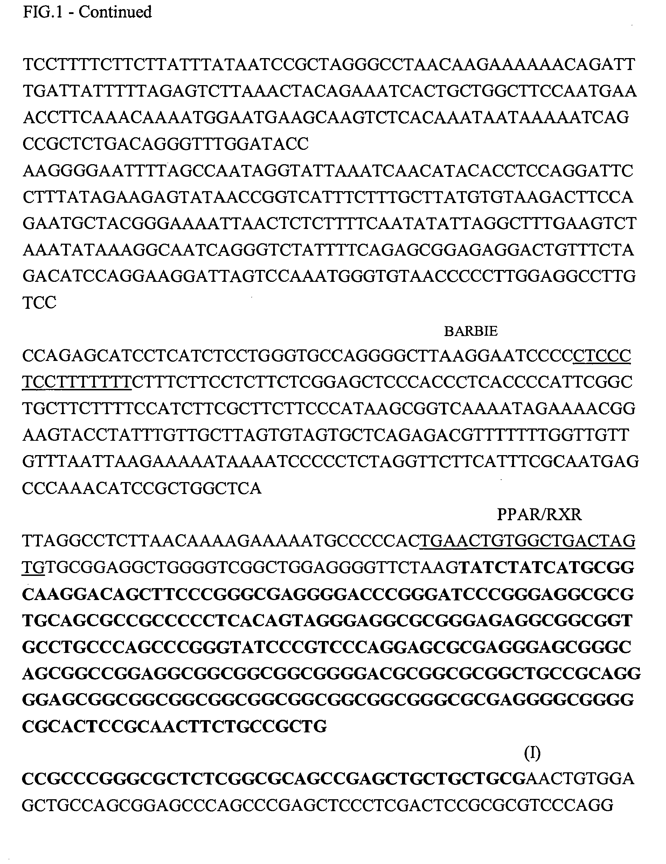

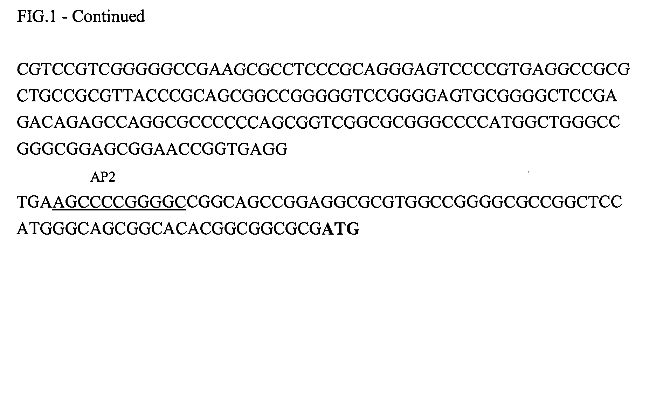

The present invention provides a substantially purified 5-HT₇ receptor gene promoter region having the nucleotide sequence shown in FIG. 1 (SEQ ID NO: 1). It further relates to the identification of transcription factors or regulatory sequence elements of the human 5-HT₇ receptor promoter, as well as to the use thereof in screening methods to identify compounds that modulate 5-HT₇ receptor gene expression.

The invention also provides a nucleic acid molecule containing a nucleotide sequence encoding a gene product operably linked to a 5-HT₇ receptor promoter region or a regulatory sequence element thereof.

The present invention further provides methods of using a gene, such as a reporter gene, operably linked to a 5-HT₇ receptor promoter or regulatory sequence element thereof in a cell-based screening assay for identifying an effective agent, that regulates the level of expression of a gene operably linked to the 5-HT₇ receptor promoter or a regulatory sequence element thereof.

Such an effective agent can be identified according to a method of the invention by introducing into the cell a nucleic acid molecule, containing the gene operably linked to the 5-HT₇ receptor promoter or the regulatory sequence element thereof; determining a control level of expression of the gene in a cell containing the gene; contacting a cell containing the gene with an agent assumed of being an effective agent; and determining a test level of expression of the gene in the cell contacted with the agent, where a difference in the test level of expression as compared to the control level of expression identifies the agent as an effective agent.

An effective agent that is identified according to a method of the invention can be, for example, a drug that reduces or inhibits 5-HT₇ receptor gene expression in a cell and, therefore, is useful in treating a disease in which presynaptic autoreceptor levels need to be controlled e.g. depression.

An effective agent can also be, for example, a drug that increases 5-HT₇ receptor gene expression and, therefore, is useful in treating a disease characterized by a reduced serotonin response.

### BRIEF DESCRIPTION OF THE DRAWINGS

Fig. 1 - Illustrates the predicted "minimal promoter" region of the human 5-HT₇ receptor (SEQ ID No: 1). Underlined sequences indicate likely transcription factor binding sites. The bold sequences indicate the GC-rich region flanking the transcription initiation site (I).

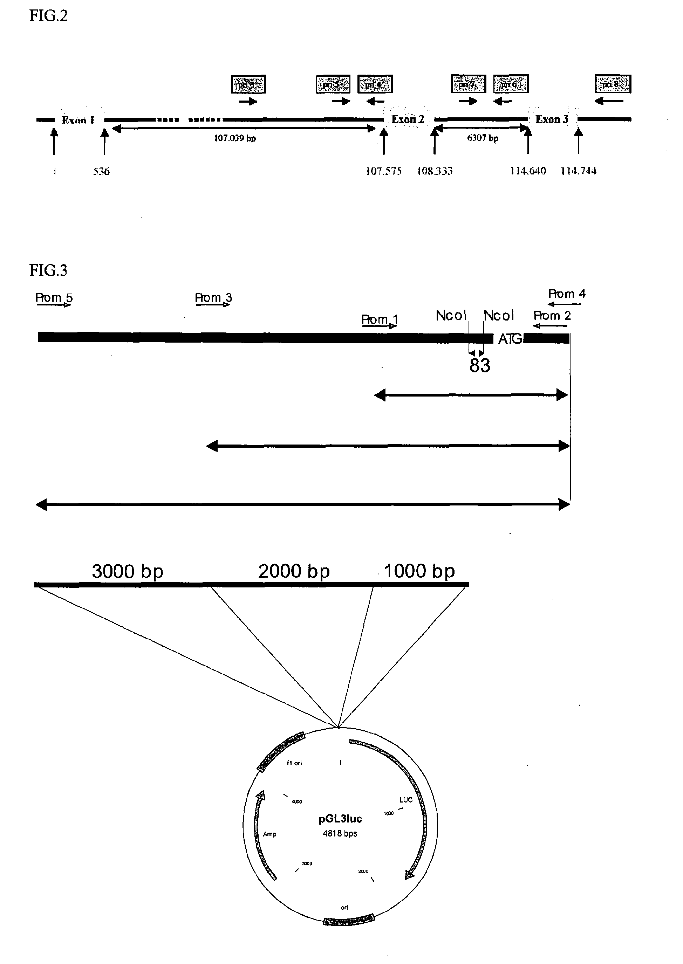

Fig. 2 - Is a map showing the structure of the human 5-HT₇ receptor gene. Exons are denoted by gray rectangles and introns by a solid line. Short horizontal arrows indicate the primers of table (I) used to check part of the intron-exon organization of the human 5-HT₇ receptor. Short vertical arrows denote the position of the intron-exon boundaries downstream of the translation initation codon ATG.
Fig. 3 - Promoter sequence primer pairs of table (II) were used to generate PCR products, which were cloned into the Zero Blunt cloning vector pCR 2.1 TOPO (Invitrogen); afterwards, the cloned fragments were sized by NcoI/HindIII digestion for the 1014 bp fragment and NcoI/XhoI digestion for the 2027 and 3011 bp fragments, respectively, and finally recloned in a similarly digested pGL3-basic luciferase reporter vector (Promega). The resultant plasmids were designated 1000luc, 2000luc and 3000luc, respectively.

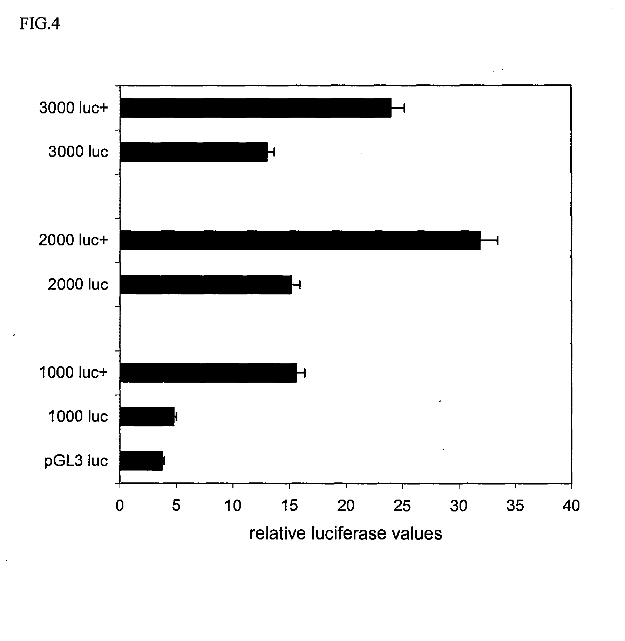

Fig. 4 - is a graph showing the relative luciferase values in HEK293T cells after transient transfection with 150 ng of the various promoter constructs. The luc+ constructs contain the 83 bp enhancer fragment (SED ID No:2).

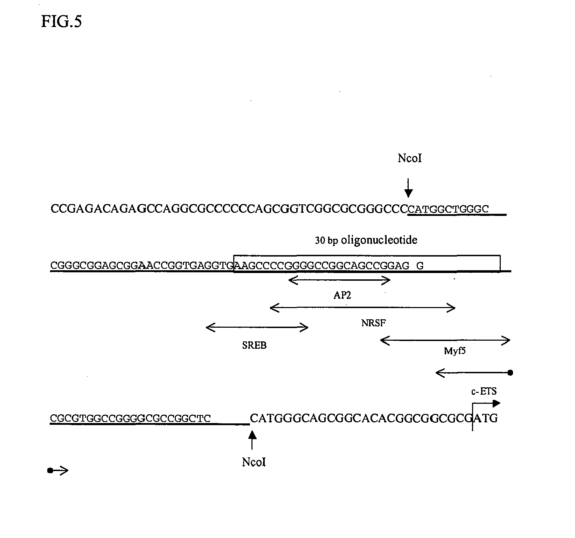

Fig. 5 - is a fragment of the human 5-HT₇ receptor promoter sequence (SEQ ID No:5) just upstream of the translation initiation codon ATG. The underlined sequence is the 83 bp NcoI-fragment (SEQ ID No:2). The boxed sequence is a 30 bp oligonucleotide sequence (SEQ ID No:4) comprising the AP2-binding motif, as indicated by a horizontal arrow. Potential binding motifs for the transcription factors NRSF, SREB, Myf5 and c-ETS are also indicated.

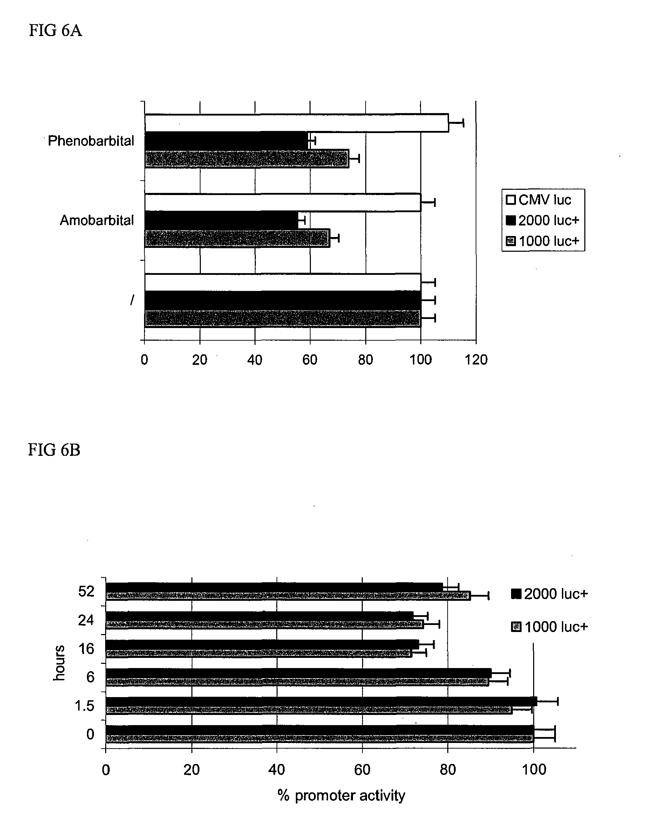

Fig. 6A - is a graph showing the influence of 1.5 mM of Amobarbital or Phenobarbital on the relative luciferase values in HEK293T cells after transient transfection with 150 ng of the 20001uc+ and 10001uc+ promoter constructs. CMVluc was used as a control vector. Luciferase activity was measured in cells treated with said barbiturates 24 hours after transfection during a period of 24 hours.
Fig. 6B - is a graph showing a time course experiment on HEK293T cells transiently transfected with 150 ng of the vectors 20001uc+ and 10001uc+ and treated with 1.5 mM Phenobarbital for induction times ranging from 1.5h till 52h.

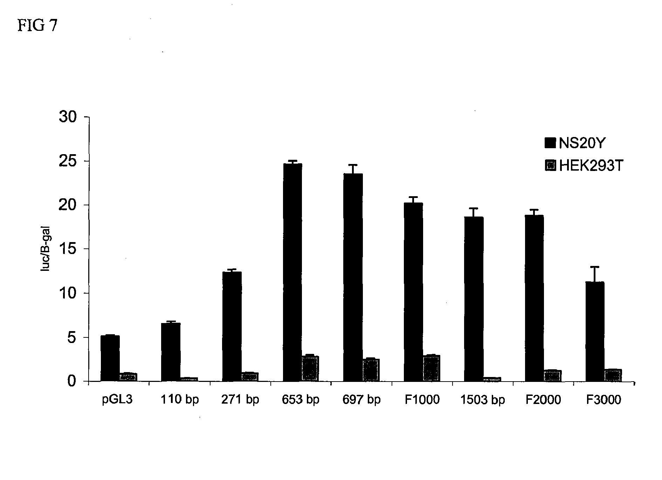

Fig. 7 - is a graph showing the relative luciferase values in HEK293T and NS20Y cells after transient transfection with 150 ng of 5-HT7 promoter constructs with different lenghts.

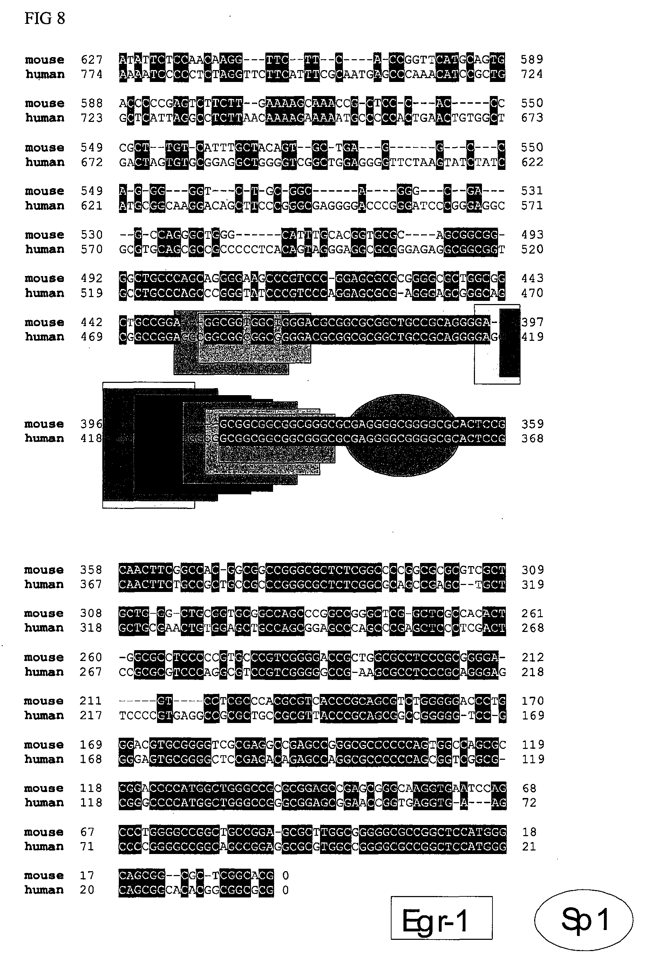

Fig. 8 - is an alignment of the human and mouse 5' UTR region in which promoter activity increased the most (271bp - 653bp). Rectangular and oval boxes indicate putative binding places of Egr-1 and Spl/MAZ respectively.

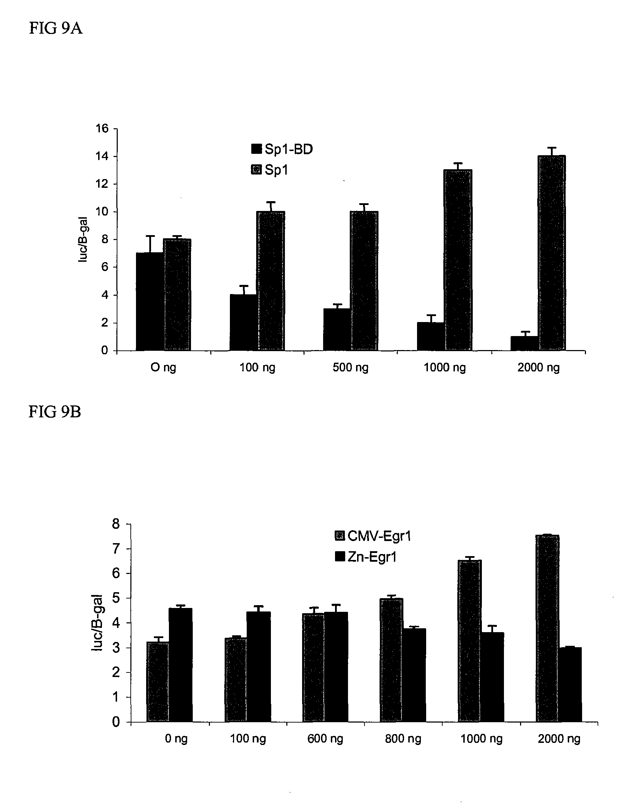

Fig. 9A - is a graph showing the relative luciferase values in NS20Y cells transfected with the GC-rich region 2331uc and different concentrations of the transcription factor CMV-Sp1 (grey bars). Different concentrations of the dominant negative construct consisting of the Sp1 binding domain (Sp1-BD) show a negative effect on 5-HT7 promoter activity (black bars).
Fig. 9B - is a graph showing the relative luciferase values in NS20Y cells transfected with the GC-rich region 2331uc and different concentrations of the transcription factor CMV-Egr-1 (grey bars). Different concentrations of the dominant negative construct consisting of the Zn-Egr-1 (Zn-Egr-1 consists of the Zn-finger domain of Egr1) show a negative effect on 5-HT7 promoter activity (black bars).

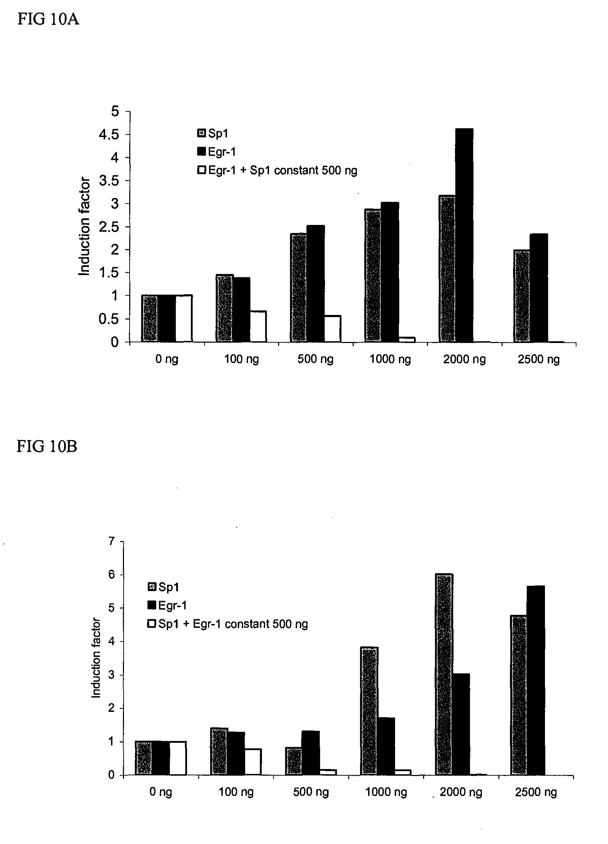

Fig. 10A - is a graph showing the relative luciferase values in NS20Y cells transfected with the F1000 luc construct and different concentrations of the transcription factors Sp1 and/or Egr-1. When Sp1 is kept constant at 500ng, an increasing concentration of Egr-1 has a negative effect on the promoter activity (white bars)
Fig. 10B - is a graph showing the relative luciferase values in NS20Y cells transfected with the F1000 luc construct and different concentrations of the transcription factors Sp1 and/or Egr-1. When Egr-1 is kept constant at 500ng, an increasing concentration of Sp1 has a negative effect on the promoter activity (white bars)

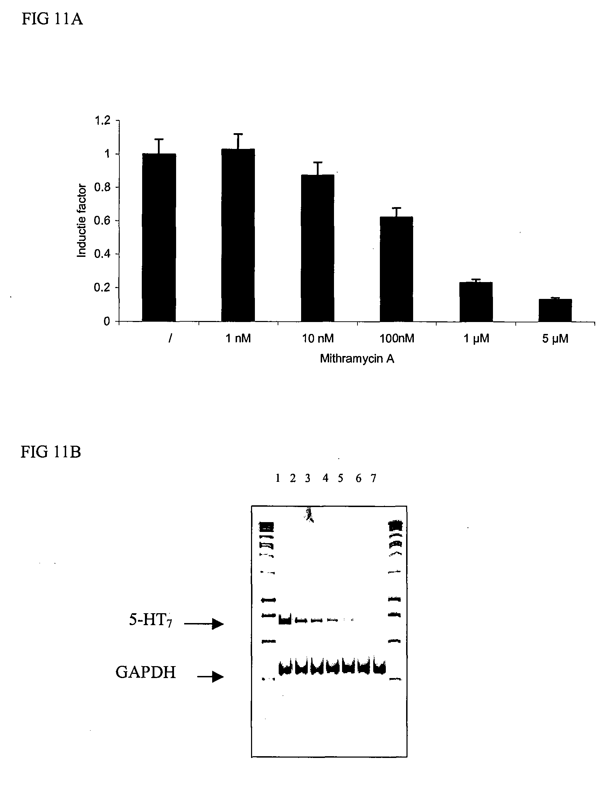

Fig. 11A - is a graph showing the relative luciferase values in NS20Y cells transfected with the F1000 luc construct and treated with different concentrations of mithramycin A for 24 h.
Fig. 11B - shows the effect of mithramycin A (lane (1) -, lane (2) 10nM, lane (3) 100 nM, lane (4) 500 nM, lane (5) 1µM, lane (6) 2 µM, lane (7) 5µM) on the endogenous 5-HT7 mRNA level of a human astrocytoma cell line 1321-N1.

### DETAILED DESCRIPTION

The present invention relates to the nucleic acid molecules constituting the human 5-HT₇ receptor promoter consisting of nucleotides 1 to 3081 of SEQ ID No:1 and fragments of said promoter having 5-HT₇ receptor promoter activity. In this previously unknown region of the gene, differentially conserved regions, consistent with regulatory function, hereinafter referred to as "regulatory elements" or "regulatory sequence elements" have been identified. Said sequences, also known as responsive elements are characterized, in part, in that they regulate expression of a linked gene and are activated due to the binding or release of a transcription factor. Numerous examples of regulatory elements and transcription factors are disclosed herein or otherwise known in the art.

The 5-HT₇ receptor promoter disclosed herein contains for example, a regulatory enhancer, hereinafter referred to as the 83 bp NcoI-fragment. The sequence shown as positions **-27** to **-110** (SEQ ID No:2) of the 5-HT₇ receptor promoter sequence (Fig 1) has a positive effect on the regulatory activity of the promoter sequence. In a further embodiment the present invention provides an AP2 binding motif at position **-50** to **-80** (SEQ ID No.:4) of the 5-HT7 promoter sequence of Figure 1.

It further contains a barbiturate-inducible element, hereinafter refered to as BARBIE. This sequence shown as position **-925** to **-939** (SEQ ID No:3) of the 5-HT₇ receptor promoter sequence was shown, in transient transfection assays, to repress 5-HT₇ receptor promoter activity in the presence of barbiturates such as Phenobarbital and Amobarbital.

It further contains a GC-rich motif. This sequence identified as position **-404** to **-637** (SEQ ID No: 6) of the 5-HT7 promoter was shown to possess 5-HT7 promoter regulation activity under influence of the transcription factors Egr-1 and Sp1.

"Negative nucleotide positions" as refered to in this description, are nucleotide positions localized within the 5-HT₇ receptor promoter region (Fig.1) upstream of the ATG start codon for translation, wherein A equals position 1 and the preceding nucleotide equals position -1.

Modulation of the 5-HT₇ receptor regulation is expected to have therapeutic value in schizophrenia, depression, affective disorders and disregulation of sleep, migraine, IBS and gastrointestinal functions.

Consequently, in a first aspect this invention provides an isolated human 5-HT₇ receptor promoter region comprising a sequence selected from:
(a) the nucleotides 1 to 3081 of SEQ ID No:1, the nucleotide sequence set forth as positions -110 to -1124 (borders of the 1000luc construct), set forth as positions -27 to -1124 (borders of the 1000luc+ construct), set forth as positions -1 to -1124 (borders of the F1000luc construct),set forth as positions -110 to -2137 (borders of the 2000luc construct), set forth as positions -27 to -2137 (borders of the 2000luc+ construct), set forth as positions, set forth as positions -1 to -2137 (borders of the F2000luc construct), set forth as positions -110 to -3081 (borders of the 3000luc construct), set forth as positions -27 to -3081 (borders of the 3000luc+ construct), set forth as positions -27 to -3081 (borders of the F3000luc construct) or as position **-404** to **-637** (borders of the 233luc construct) in SEQ ID NO: 1, or a fragment thereof exhibiting 5-HT₇ receptor promoter activity;
(b) the complementary strand of (a); and
(c) nucleotide sequences capable of hybridizing, under stringent hybridization conditions, to a nucleotide sequence as defined in (a) or (b).

"Promoter region" refers to a region of DNA that functions to control the transcription of one or more genes, and is structurally identified by the presence of a binding site for DNA-dependent RNA polymerase and of other DNA sequences on the same molecule which interact to regulate gene transcription.

By "isolated" in reference to a nucleic acid is meant a polynucleotide, including RNA or DNA that is isolated from a natural source or that is synthesized. The isolated nucleic acids of the present invention are unique in the sense that they are not found in a pure or separated state in nature. Use of the term "isolated" indicates that a naturally occurring sequence has been removed from its normal cellular context. Thus, the sequence may be in a cell-free solution or placed in a different cellular environment or nucleic acid context.

"Stringent" hybridization conditions are hybridization in 6X SSC at about 45°C, followed by one or more washes in 0.2X SSC, 0.1%SDS at 65°C.

Another aspect of the invention is a recombinant DNA molecule comprising the human 5-HT₇ receptor promoter region as defined above. In the said recombinant DNA molecule, the human 5-HT₇ receptor promoter region can be operably linked to a nucleic acid molecule encoding a detectable product, such as the human 5-HT₇ receptor gene, or a reporter gene. The term "operably linked", as used herein, means functionally fusing a promoter with a gene in the proper frame to express the gene under control of the promoter. As used herein, the term "reporter gene" means a gene encoding a gene product that can be identified using simple, inexpensive methods or reagents and that can be operably linked to the human 5-HT₇ receptor promoter region or an active fragment thereof. Reporter genes such as, for example, a firefly luciferase, β-galactosidase, alkaline phosphatase, the bacterial chloramphenicol acetyl transferase or green fluorescent protein reporter gene, can be used to determine transcriptional activity in screening assays according to the invention (see, for example, Goeddel (ed.), Methods Enzymol., Vol. 185, San Diego:Academic Press, Inc. (1990); see also Sambrook, supra). In a preferred embodiment, the reporter gene is the firefly luciferase gene or the green fluorescent protein.

The invention also provides a vector comprising the recombinant DNA molecule as defined above, as well as a host cell stably transformed with such a vector, or generally with the recombinant DNA molecule according to the invention. The term "vector" refers to any carrier of exogenous DNA that is useful for transferring the DNA into a host cell for replication and/or appropriate expression of the exogenous DNA by the host cell. Accordingly, in a specific embodiment said vector is an expression vector such as pGL3luc, pBLCAT5 (LMBP 2451), pGMCSFlacZ (LMBP 2979), pEGFP or pSEAPbasic (DMB 3115),wherein LMBP and DMB numbers refer to the accession numbers of these expression vectors at the Belgian Co-ordinated Collections of Microorganisms.

In another aspect, the invention provides a method for identification of a compound modulating 5-HT₇ receptor promoter activity, said method comprising the steps: (i) contacting a candidate agent with a human 5-HT₇ receptor promoter region as defined above; and (ii) determining whether said candidate agent modulates expression of the detectable product, such modulation being indicative for an agent capable of regulating 5-HT₇ receptor promoter activity. The detectable product refers either to the 5-HT₇ receptor protein or to the product of a reporter gene such as luciferase, β-galactosidase or green fluorescent protein. Methods to quantify the detectable products are generally known in the art and include amongst others the use of a colorimetric substrate if the expression product is an enzyme, the use of specific antibodies in an RIA or ELISA assay or the measurement of the level of mRNA transcribed from genes operably linked to the promoter, wherein said mRNA can be measured either directly or indirectly using standard procedures.

As used herein, the term "compound" or "agent" means a biological or chemical compound such as a simple or complex organic molecule, a peptide, a protein or an oligonucleotide. A "test compound" as used herein, refers to a "compound" or "agent" used in a method according to the invention to assess whether said compounds modulates 5-HT₇ receptor promoter activity or interacts with "regulatory elements" thereof.

A transfection assay can be a particularly useful screening assay for identifying an effective agent modulating and/or regulating 5-HT₇ receptor promoter activity. In a transfection assay, a nucleic acid encoding a gene, e.g. a reporter gene, operably linked to a human 5-HT₇ receptor promoter or an active fragment thereof, is transfected into the desired cell type. A test level of reporter gene expression is assayed in the presence of a candidate agent and compared to a control level of expression. An effective agent is identified as an agent that results in a test level of expression that is different than a control level of reporter gene expression, which is the level of expression determined in the absence of the agent.

Methods for transfecting cells and a variety of convenient reporter genes are well known in the art (see, for example, Goeddel (ed.), Methods Enzymol., Vol. 185, San Diego: Academic Press, Inc. (1990); see also Sambrook, supra). Consequently, the said method could e.g. comprise assaying reporter gene expression in a host cell, transiently or stably transformed with a recombinant DNA molecule comprising the human 5-HT₇ receptor promoter, in the presence and absence of a candidate agent, wherein an effect on the test level of expression as compared to control level of expression is indicative of an agent capable of regulating 5-HT₇ receptor promoter activity. A specific embodiment, wherein different 5-HT₇ receptor promoter fragments are linked to the firefly luciferase, is provided in the Experimental procedures.

Methods for identification of polypeptides regulating 5-HT₇ receptor promoter activity could include various techniques known in the art, such as electrophoretic mobility shift assay (EMSA) to identify proteins that bind to the 5-HT₇ receptor regulatory region (Dignam D. et al. (1983) Nucl. Acids Research 11, 1475-1489); such as the yeast one-hybrid system (see: Li & Herskowitz (1993) Science 262, 1870-1874) to identify proteins binding specific sequences from the 5-HT₇ receptor regulatory region; biochemical purification of proteins which bind to the regulatory region, the use of a "southwestern" cloning strategy (see e.g. Hai et al. (1989) Genes & Development 3: 2083-2090), in which a pool of bacteria infected with a "phage library" are induced to express the encoded protein and probed with radioactive DNA sequences from the 5-HT₇ receptor regulatory regions to identify binding proteins.

In a further aspect, the invention provides isolated regulatory elements of the 5-HT₇ receptor promoter region comprising a sequence selected from:
(a) the nucleotide sequence set forth as positions **-27** to **-110** (SEQ ID No:2) in SEQ ID No: 1, or a fragment thereof exhibiting 5-HT₇ receptor enhancer activity;
(b) the nucleotide sequence set forth as positions **-925** to **-939** (SEQ ID No:3) in SEQ ID No: 1, or a fragment thereof exhibiting barbiturate-inducible element activity;
(c) the nucleotide sequence set forth as positions **-50** to **-80** (SEQ ID No:4) in SEQ ID No: 1, or a fragment thereof exhibiting 5-HT₇ receptor promoter regulation activity;
(d) the nucleotide sequence set forth as positions **-404** to **-637** (SEQ ID No:6) in SEQ ID No: 1, or a fragment thereof exhibiting 5-HT₇ receptor promoter regulation activity;
(e) the complementary strand of (a), (b), (c) or (d); and
(f) nucleotide sequences capable of hybridizing, under stringent hybridization conditions, to a nucleotide sequence as defined in (a), (b), (c)or (d).

As described above for the 5-HT₇ receptor promoter region, the invention further provides a recombinant DNA molecule comprising one of the aforementioned regulatory elements, operably linked to a nucleic acid molecule that encodes a detectable product. Said detectable product could either be the 5-HT₇ receptor polypeptide or a reporter gene, such as the firefly luciferase gene, alkaline phosphatase, the bacterial chloramphenicol acetyl transferase gene, the green fluorescent protein gene or the β-galactosidase gene. A regulatory element that lacks minimal promoter elements, and as such cannot regulate expression of the linked gene, can be operably linked to a reporter plasmid containing a minimal promoter, such as the minimal interleukin 6 (IL6) promoter (phu.IL6P-50luc+ - Plaisance et al. (1997) MCB 17, 3733-3743), the minimal E1B promoter (pMCSLuc - Stratagene) or the TK promoter for luciferase (pTKLuc - Promega). In a preferred embodiment the recombinant molecule comprises an isolated nucleic acid molecule selected from the group consisting of the 83 bp NcoI-fragment (SEQ ID No:2), the BARBIE element (SEQ ID No:3) or the 233bp GC-rich motif (SEQ ID No:6).

Further, the invention provides a method for identification of a compound modulating or interacting with 5-HT₇ receptor promoter regulatory element activity, said method comprising the steps: (i) contacting a test compound with the human 5-HT₇ receptor promoter regulatory element as defined above; and (ii) determining whether said candidate agent modulates expression of the 5-HT₇ receptor gene, such modulation being indicative for an agent capable of modulating or interacting with 5-HT₇ receptor promoter regulatory element activity.

Accordingly the invention provides an isolated human 5-HT₇ receptor enhancer region comprising a sequence selected from:
(a) the nucleotide sequence set forth as positions **-27** to **-110** (SEQ ID No:2) in SEQ ID NO: 1, or a fragment thereof exhibiting 5-HT₇ receptor enhancer activity;
(b) the nucleotide sequence set forth as positions **-404** to **-637** (SEQ ID No:6) in SEQ ID NO: 1, or a fragment thereof exhibiting 5-HT₇ receptor enhancer activity;
(c) the complementary strand of (a) or (b); and
(d) nucleotide sequences capable of hybridizing, under stringent hybridization conditions, to a nucleotide sequence as defined in (a), (b) or (c).
"Enhancer region" refers to a region of DNA that functions to control the transcription of one or more genes.

As described above for the human 5-HT₇ receptor promoter region, the invention further provides a recombinant DNA molecule comprising a human 5-HT₇ receptor enhancer region, a vector comprising the said recombinant DNA molecule, as well as a host cell stably transformed with said vector or with said recombinant DNA molecule.

Further, the invention provides a method for identification of a compound modulating 5-HT₇ receptor promoter activity, said method comprising the steps: (i) contacting a test compound with the human 5-HT₇ receptor enhancer region as defined above; and (ii) determining whether said test compound modulates expression of the 5-HT₇ receptor gene, such modulation being indicative for an agent capable of regulating 5-HT₇ receptor promoter activity. It will be understood by the skilled person that known steps are available for performing such a method. For instance, a "panel" of constructs which include a variety of mutations and deletions can be used in order to associate a response with a specific alteration of a single base or sub segment of the regulatory apparatus. A simple panel might include: enhancer plus promoter, promoter only, enhancer plus a "minimal" promoter from a distinct gene.
As mentioned above, a transfection assay, using a host cell stably transformed with a suitable recombinant DNA molecule, can be a particularly useful screening assay for identifying an effective agent.

A further aspect of the invention is a vector comprising the nucleic acid molecules according to the invention. The said vector can e.g. be a replicable expression vector, which carries and is capable of mediating the expression of a DNA molecule according to the invention. In the present context the term "replicable" means that the vector is able to replicate in a given type of host cell into which is has been introduced. Examples of vectors are viruses such as bacteriophages, cosmids, plasmids and other recombination vectors. Nucleic acid molecules are inserted into vector genomes by methods well known in the art.

Included in the invention is also a cultured host cell harboring a vector according to the invention. Such a host cell can be a prokaryotic cell, a unicellular eukaryotic cell or a cell derived from a multicellular organism. The host cell can thus e.g. be a bacterial cell, such as an *E. coli* cell; a yeast cell, such as *Saccharomyces cerevisiae* or *Pichia pastoris,* or a mammalian cell, such as HEK293 cells. The methods employed to effect introduction of the vector into the host cell are standard methods, well known to a person familiar with recombinant DNA methods. Preferred cells as exemplified below consist of HEK293 cells, NS20Y cell or N2A cells.

It is thus an object of the present invention to provide a method for identifying compounds capable of modulating 5HT₇ receptor promoter activity said method comprising: (a) contacting a host cell comprising a recombinant DNA molecule according to the invention with a test compound; and (b) determining whether said test compound modulates the level of expression of the detectable product. The recombinant DNA molecule in the aforementioned method preferably comprises an isolated nucleic acid molecule selected from;
(a) the nucleotide sequence set forth as positions **-27** to **-110** (SEQ ID No:2) in SEQ ID No: 1, or a fragment thereof exhibiting 5-HT₇ receptor enhancer activity;
(b) the nucleotide sequence set forth as positions **-925** to **-939** (SEQ ID No:3) in SEQ ID No: 1, or a fragment thereof exhibiting barbiturate-inducible element activity; or
(c) the nucleotide sequence set forth as positions **-404** to **-637** (SEQ ID No:6) in SEQ ID No: 1, or a fragment thereof exhibiting 5-HT₇ receptor promoter regulation activity.

Accordingly the present invention provides;
A method for identifying compounds that modulate 5HT₇ receptor promoter enhancer activity, said method comprising:
(a) contacting a host cell transfected with a recombinant DNA molecule comprising the 83 bp NcoI-fragment (SEQ ID No:2) operably linked to a detectable product, with a test compound, and
(b) determining whether said test compound modulates the level of expression of the detectable product.
A method for identifying compounds that modulate the activity of the barbiturate-inducible element within the 5HT₇ receptor promoter region, said method comprising:
(a) contacting a host cell transfected with a recombinant DNA molecule comprising the BARBIE element (SEQ ID No:3) operably linked to a detectable product, with a test compound, and
(b) determining whether said test compound modulates the level of expression of the detectable product.
A method for identifying compounds that modulate the activity of the barbiturate-inducible element within the 5HT₇ receptor promoter region, said method comprising:
(a) contacting a host cell transfected with a recombinant DNA molecule comprising the GC-rich motif (SEQ ID No:6) operably linked to a detectable product, with a test compound, and
(b) determining whether said test compound modulates the level of expression of the detectable product.

In an alternative embodiment the aforementioned assay consists of a binding assays wherein the transcription factors are used as specific ligands for the regulatory elements of the 5HT7 receptor promoter. It is thus an object of the present invention to provide a method for identifying compounds capable of modulating the 5HT₇ receptor promoter enhancer activity, said method comprising; (a) contacting a recombinant DNA molecule comprising the regulatory elements of the 5-HT7 receptor promoter according to the invention with a test compound in the presence of a transcription factor, and (b) determining whether said test compound affects the binding of said transcription factor to the regulatory element.

In particular the present invention provides a method for identifying compounds capable of modulating the 5HT₇ receptor promoter enhancer activity, said method comprising; (a) contacting a recombinant DNA molecule comprising the 83 bp NcoI-fragment (SEQ ID No:2) with a test compound in the presence of the transcription factor AP2, and (b) determining whether said test compound affects the binding of said transcription factor to the 83 bp enhancer region (SEQ ID No:2).

In a further particular embodiment the present invention provides a method for identifying compounds capable of modulating the 5HT₇ receptor promoter enhancer activity, said method comprising; (a) contacting a recombinant DNA molecule comprising the GC-rich motif (SEQ ID No:6) with a test compound in the presence of the transcription factor Sp1 or Egr-1, and (b) determining whether said test compound affects the binding of said transcription factor to the GC-rich motif (SEQ ID No:6).

Methods to determine binding of the transcription factor to its recognition site are known in the art and comprise for example the gel mobility shift assay as outlined in the experimental part hereinafter.

In yet another aspect, the invention includes a method for identifying an agent capable of regulating expression of the nucleic acid molecule as defined above, said method comprising the steps (i) contacting a candidate agent with the said nucleic acid molecule; and (ii) determining whether said candidate agent modulates expression of the said nucleic acid molecule.

Antisense nucleic acids or siRNAs (preferably 10 to 25 base-pair oligonucleotides) capable of specifically binding to control sequences for the 5-HT₇ receptor gene are introduced into cells, e.g. by a viral vector or a colloidal dispersion system such as a liposome. The antisense nucleic acid or siRNAs binds to a target nucleotide sequence in the cell and prevents transcription and/or translation of said target sequence. Phosphorothioate and methylphosphonate antisense oligonucleotides are specifically contemplated for therapeutic use by the invention. Suppression of expression of the 5-HT₇ receptor gene, at either the transcriptional or translational level, is useful to generate cellular or animal models for diseases/conditions related to serotonin mediated responses.

In yet another aspect, the invention provides a method for the identification of polypeptides, which bind to nucleotide sequences involved in the biological pathway related to serotonin-mediated responses, comprising the steps of:
(a) transfecting a host cell line with a human 5-HT₇ receptor promoter nucleotide sequence linked to a reporter gene, such as a gene encoding Green Fluorescent Protein (GFP) (for a review, see e.g. Galbraith et al. (1999) Methods in Cell Biology 58: 315-341);
(b) transfecting the said host cell line with a variety of human cDNA sequences, e.g. sequences included in a cDNA library;
(c) identifying and isolating cells, e.g. by FACS cells sorting, having an altered level of expression of the said reporter gene, which is indicative that the polypeptide encoded by the added cDNA up- or downregulates at least one gene involved in the biological pathway related to serotonin-mediated responses;
(d) recovering cDNA from the cells isolated in step (c), by standard procedures, e.g. PCR or a CRE-LOX mediated procedure (see e.g. Sauer (1998) Methods 14: 381-392); and
(e) identifying the polypeptide expressed by the cDNA recovered in step (d), e.g. by sequencing the cDNA and comparing the obtained sequence against sequence databases.

Throughout this description the terms "standard methods", "standard protocols" and "standard procedures", when used in the context of molecular biology techniques, are to be understood as protocols and procedures found in an ordinary laboratory manual such as: Current Protocols in Molecular Biology, editors F. Ausubel et al., John Wiley and Sons, Inc. 1994, or Sambrook, J., Fritsch, E.F. and Maniatis, T., Molecular Cloning: A laboratory manual, 2nd Ed., Cold Spring Harbor Laboratory Press, Cold Spring Harbor, NY 1989.

This invention will be better understood by reference to the Experimental Details that follow, but those skilled in the art will readily appreciate that these are only illustrative of the invention as described more fully in the claims, which follow thereafter. Additionally, throughout this application, various publications are cited. The disclosure of these publications is hereby incorporated by reference into this application to describe more fully the state of the art to which this invention pertains.

### EXPERIMENTAL PROCEDURES

### Identification of the human 5-HT₇ receptor genomic clone

The known cDNA sequence of the human 5-HT₇ serotonin receptor (GI:17864131) was used to search the NCBI-database (http://www.ncbi.nlm.nih.gov/BLAST/). We identified a BAC clone (GI:14547262): Human DNA sequence from RP11-103A2 on chromosome 10, complete sequence, which contains the complete coding sequence of the 5-HT₇ receptor. Based on the BAC sequence information, primers were designed to confirm the presence of 5-HT₇ exons in the BAC clone obtained from the Sanger Centre. Sequences of the primers are shown in table I.
After an initial denaturing step (94°C for 5 min), PCR was performed for 25 cycles (denaturing step of 94°C for 1 min, annealing step of 59°C for 1 min and an extension step of 68°C for 10 min) using Pfu DNA polymerase (Promega).
Recloning into the EBV-based vector was performed by Richard Wade-Martins using the Cre-LoxP technique.

### Promoter constructs and plasmids

Based on the BAC sequence, primers were designed to generate three different lengths of the 5'flanking region (Table II).
All fragments were generated using Pfu polymerase according to the following protocol. Thirty cycles of PCR were done at 94°C for 1 min, 48°C for 1 min and 72°C for 4 min.
The obtained PCR products were cloned into the Zero Blunt cloning vector pCR 2.1 TOPO (Invitrogen) and several clones were verified by sequence analysis.
For subcloning, a NcoI/HindIII digested 1014 bp fragment or NcoI/XhoI digested 2027 and 3011 bp fragments were ligated to a similarly digested pGL3-basic luciferase reporter vector (Promega). The resultant plasmids were designated 1000 luc, 2000 luc and 3000 luc, respectively (Fig. 3). Due to this cloning strategy all constructs lack 110 bp directly upstream of translation start point. A new fragment of 83 bp was successfully subcloned in all three reporter constructs, resulting in the plasmids 1000 luc+, 2000 luc+ and 3000 luc+. The orientation of the inserted 83 bp was verified by sequencing.
pGL3-basic, which lacks a promoter and enhancer region, and CMV luc, in which luciferase is under control of the strong CMV promoter, were used as negative and positive control, respectively. pNeogal was used to correct for variation in transfection efficiency.

A linker sequence of the same identity as the 27 bp missing in the plasmids 1000 luc+, 2000 luc+ and 3000 luc+ was inserted at the correct position and orientation in all 3 plasmids generating the constructs F1000 luc, F2000 luc and F3000 luc. The 233 bp luc fragment, hereinafter refered to as the 233luc construct, was generated by cutting the 233 bp out of the F1000 luc construct with BamHI/BglI. After Klenow-treatment, the obtained 233 bp was cloned into a NcoI opened and blunted pGL3 luc vector and the sequence and orientation was confirmed by sequence analysis.

### Cell culture and transient expression assays

HEK293T cells were maintained in Dulbecco's modified Eagle's medium supplemented with 100 units/ml penicillin, 100 µg/ml streptomycin, and 10% newborn calf serum. These cells were maintained at 37°C in a humidified atmosphere of 5% CO₂.
10⁵ cells/cm² in 24-well dishes were transfected with 150 ng promoter plasmid and 50 ng neogal (served as an internal control), using the standard calcium phosphate coprecipitation method. 24 hours after transfection, cells were washed once with PBSA and lysed in 150 µl 100 mM potassium phosphate (pH 7.8), 0.2% Triton X-100. The samples were analyzed for luciferase activity as described previously (Vanden Berghe et al., 1998). β-Galactosidase activity was measured using Galacto Star (Tropix) as substrate. For the barbiturate experiments, treatment with different barbiturates started 24 hours after transfection of the cells
For the CMV-AP2 overtransfection experiments, 3 x 10⁶ HEK293T cells were transfected with 12 µg CMV-AP2 plasmid (gift R. Tjian) using the calcium phosphate method.
N2A and NS20Y cells were maintained in Dulbecco's modified Eagle's medium supplemented with 100 units/ml penicillin, 100µg/ml streptomycin, and 10% foetal calf serum. These cells were maintained at 37°C in a humidified atmosphere of 5% CO₂. 4x10⁵ cells in 6 well dishes were transfected with 500 - 1000 ng promoter construct and 300 ng neogal (served as internal control), using JetPEI a cationic polymer transfection reagent (Qbiogene).
In the co-transfection experiments with Egr-1, Zn-Egr-1, Sp1 and BD-Sp1, 4x10⁵ NS20Y or N2A cells were transfected with JetPEI and the amounts of DNA as indicated in the figures (always correcting the total amount of DNA to equal levels in each well with pcDNA3).
Cells were treated with different concentrations of Mithramycin A for 24 hours.

### Gel mobility shift assay

Total extracts of the different cell lines were prepared with lysis buffer as previously described. The 83 bp probe was isolated from the TOPO 2138 construct by NcoI digestion. The 30 bp sequence was generated by annealing 5'-GAGGTGAAGCCCCGGGGCCGG CAG-3' and 5'-CCGGTGCCGGCCCCGGGGCTTCA-3' according to the following scheme: 94 °C for 10 min, 70 °C for 10 min, 50 °C for 10 min, 30 °C for 20 min and 4 °C for 10 min. The DNA fragments were end-labeled with [α-³²P]dCTP using Klenow DNA polymerase (Promega). Binding reactions were carried out in a final volume of 20 µl containing 50 mM Hepes (pH 7.9), 375 mM KCl₂, 12.5 mM MgCl₂, 0.5 mM EDTA, 5 mM DTT and 15% Ficoll. Cell extracts were preincubated in the reaction buffer for 10 min on ice, after which the labeled DNA probe was added, and incubation continued for another 15 min at room temperature.
In competition experiments an excess of unlabeled 30 bp probe was added prior to the addition of the labeled 83 bp probe. Electrophoresis was performed using 6% polyacrylamide gels in 0.5x TBE for 2 hours. Results were analyzed using a Biorad Molecular Imager.

### Identification of transcription initiation site of the human 5-HT₇ receptor gene

5' RACE was performed using the RLM-RACE technique according to the manufactures protocol (Ambion). 10 µg of total human fetal brain RNA (Stratagene) was treated with calf intestinal phosphatase (CIP) (Ambion) to remove free 5'phosphates from molecules such as ribosomal RNA, fragmented mRNA, tRNA and contaminating genomic DNA. After phenol:chloroform (Invitrogen) extraction, RNA was precipitated and resolved in 11 µl nuclease-free water. 5 µl was treated with 2 µl tobacco acid pyrophosphatase (TAP) (Ambion) in a total of 20 µl for one hour at 37°C. TAP removes the cap structure from full-length mRNA, leaving a 5'monophosphate. Next, a 45 base RNA adaptor oligonucleotide (5'-GCUGAUGGCGAUGAAUGAACACUGCGUUUGCUGGCUUUGAUGAAA-3') was ligated to the RNA population using 5 µl CIP/TAP-treated RNA, 1 µl 5' RACE adaptor and 2 µl T4 RNA ligase (Ambion) in a total volume of 20 µl for one hour at 37°C. The ligated RNA was then used in a reverse transcriptase reaction with random primers (Ambion) and M-MLV reverse transcriptase (Ambion) for one hour at 42°C. Using 1 µl of this cDNA, three successive nested PCR reactions were performed. The first PCR reaction was performed with an adaptor-specific outer primer (5'-GCTGATG GCGATGAATGAACACTG-3') and a gene-specific primer B 1 (5'-GGTGGTGGCTGCTTTCTGTTCTCGCTTAAA-3'). The PCR reaction was performed in a final volume of 50 µl under following conditions: 94°C for 30 sec, 60°C for 30 sec, and 72°C for 1 min (35 cycles). One-tenth of this PCR mixture was then amplified further in a second nested PCR reaction using an adaptor-specific outer primer (see above) and gene-specific outer primer (5'-ATGACGTCCATGGCGATGAA-3') under the same conditions as above. Finally one-tenth of this PCR amplified mixture was used in a third nested PCR using an adaptor-specific inner primer (5'-CGCGGATCCGAACACTGCGTTTGCTGGCTTTGATG-3') and gene-specific inner primer (5'-GATGGAGCCGATCACAACTT-3') applying again the same PCR conditions. 5 µl of the final PCR reaction was cloned into the TOPO TA cloning vector pCR 2.1 TOPO (Invitrogen) and positive clones were verified by sequencing.

### RT-PCR analysis of endogenous 5-HT7 mRNA levels

132-1N1 human astrocytoma cells were seeded into 58 cm² plates; 12 hours after seeding the cells were cultured with either medium alone (-) or medium containing 10 nM, 100 nM, 500 nM, 1µM, 2 µM or 5 µM Mithramycin A (Serva). Total RNA was isolated using a RNeasy, total RNA isolation kit (Qiagen). cDNA was synthesized using AMV reverse transcriptase (Promega). The cDNA was then amplified by PCR in a total volume of 30 µl containing 10 pmol of each primer, 200 µM dNTP, *Taq* DNA polymerase buffer, 2,5 units of *Taq* and 5 µl of cDNA. PCR conditions were 94°C, 30 sec; 56°C, 1min; 72°C, 2 min; for 35 cycles. PCR products were analyzed by electrophoresis through 6% polyacrylamide gel.
5-HT₇ forward primer F1: GGAACAGATCAACTACGGCAGAGT
5-HT₇ reverse primer B1: GGTGGTGGCTGCTTTCTGTTCTCGCTTAAA
Use of F1 and B1 results in a PCR product of 780 bp.
GAPDH forward primer: ACCACAGTCCATGCCATCAC
GAPDH reverse primer: TCCACCACCCTGTTGCTGTA
Use of GAPDH primers results in a PCR product of 452 bp.

### RESULTS

### Identification of the genomic clone

The published cDNA-sequence of the 5-HT₇ receptor (GI:17864131) was used to perform a full human genome blast search on the NCBI blast website
(http://www.ncbi.nlm.nih.gov/BLAST/). This resulted in the identification of a BAC clone (GI:14547262) which contains the complete genomic sequence of the human 5-HT₇ receptor. This BAC clone was ordered from the Sanger Centre
(http://www.sanger.ac.uk/) and the presence of different exon and intron sequences was identified by PCR, using the primer sets Pri 3 - Pri 4 (6643 bp), Pri 5 - Pri 6 (8597 bp) and Pri 7 - Pri 8 (6015 bp). The exon-intron organization of the human 5-HT₇ receptor was revealed by compairing cDNA sequence with that of human genomic DNA. Apart from the coding sequences and the introns, this BAC clone also contained 14.025 bp and 28.421 bp of the upstream and downstream sequences respectively. Fig. 2 shows the structure of the human 5-HT₇ receptor.
The BAC clone, containing the complete genomic insert was retrofitted into an EBV vector using the LoxP-Cre recombinase technique (Wade-Martins, 1999; Wade-Martins, 2000).
The presence of the genomic insert in the EBV vector was again confirmed by PCR using the same primer sets as for the verification of the BAC clone. This EBV-vector allows us to transfer the full genomic 5-HT₇ receptor sequence to eukaryotic cells and will therefore be useful for the identification of the sequences controlling the alternative splicing as well as to study the promoter regulation of the gene.

### Identification of the promoter region

Sequence analysis of the 5'-flanking DNA using MatInspector
(http://www.genomatix.de) reveals consensus binding sites for multiple transcription factors. The 5' flanking region (Fig.1) is GC-rich and contains no TATA box or CAAT box near the transcription start sites (see futher). Interestingly, several neuron-restrictive silencer element/repressor element 1 (NRSE/RE1) motifs were identified, which may be important for neuronal restriction of the 5-HT₇ receptor gene.
Several other motifs which might be of importance in the regulation of the 5-HT₇ gene are Sp1, AP2, OLF1, BARBIE, PPAR/RXR, NGFIC, Egr,....
When we used Proscan (http://bimas.dcrt.nih.gov/molbio/proscan) and PromoterInspector (http://www.genomatix.de) to identify a potential promoter in the 5' flanking region, both programs indicated a putative promoter region between -663 and -68 upstream of the ATG codon.

This was confirmed in transient transfection of HEK293T and NS20Y cells using different lengths of the 5HT7 promoter region (Figure 7). The region in which promoter activity increased the most (271bp - 365bp) has a GC-rich region and putative binding places of Sp1/MAZ and Egr-1 (Figure 8). This region of 233bp (SEQ ID No: 6) was cut out of the F1000 construct by restriction digest with BamHl and Bgl1 and cloned into pGL3luc giving rise to the construct 233luc.

### Analysis of the promoter region

In order to investigate potential promoter activities of the 5-HT₇ receptor gene, fragments of variable lengths were isolated using specific primer sets and high fidelity Pfu DNA polymerase (Table II). The PCR fragments were cloned in a Zero-Blunt vector pCR2.1 TOPO (Invitrogen) and from there the promoter fragments were subcloned into the pGL3-basic luciferase reporter vector (Promega).
Due to the cloning strategy all constructs initially lacked a 83 bp NcoI-fragment (SEQ ID No:2). This fragment was subsequently inserted into all promoter constructs giving rise to the '+' constructs. All constructs still miss 27 bp preceding the translational start site.
Figure 4 indicates that all fragments exhibit considerable transcriptional activity in HEK293T cells. The highest activity was obtained using a 2000 bp fragment indicating the presence of a potential enhancer element compared to the 1000 bp fragment. On the other hand the use of an even longer promoter fragment resulted in drop in expression level pointing out the presence of a potential repressor element from position -2000 to -3000. However, for all constructs, we found a distinct reduction in promoter activities when the 83 bp NcoI-fragment was not present. These results clearly indicate the presence of an important basic promoter- or enhancer element within this 83 bp sequence.
Transient transfection of different lengths of the 5-HT7 presumed promoter region, let to significant increase in promoter activity in the region between 271bp and 653 bp (Figure 7). Analysis of this region revealed several potential Egr-1 binding sites and at least one putative Sp1 binding site (Figure 8).
Using a SmarTest (http://www.genomatix.de) we could also identify the presence of a putative S/MAR sequence ranging from position 2840 untill 2590. S/MAR are believed to be master controllers of promoter activity so that they could block influence of neighboring sequences. Whether this implies that all 5-HT₇ promoter activity resides within the 3000 bp fragment will be investigated by using longer promoter fragments.

In summary, these results indicate that the fragments isolated from the 5'-untranslated region of the 5-HT₇ receptor clearly displayed promoter activity.

### Identification of transcription initiation site in the human 5-HT₇ gene

Starting from human fetal brain RNA (Stratagene) 5' RACE was performed using gene-specific antisense primers derived from the known sequence of the human 5-HT₇ receptor. After a first round with an adaptor-specific outer primer and primer B1, a 800 bp fragment could be detected. This fragment was further amplified using a second adaptor-specific outer primer and the gene-specific outer primer, resulting in a 600 bp fragment. Finally a 400 bp fragment was generated using an adaptor-specific inner primer and a gene-specific inner primer in a third round of PCR. This 400 bp fragment was cloned into TOPO-TA vector and sequence analysis indicated the presence of a transcription initiation start site 307 bp upstream of the start site of translation ATG. This transcription site differs from the published one identified by Bard (GI:10880132, GI:10880130, GI:10880128) and Heidmann (GI:1857144, GI:1857142).

### Identification of interesting potential transcription factors on the 83 bp fragment

Our analysis in HEK293T cells revealed the importance of the 83 bp NcoI-fragment for 5-HT₇ promoter activity. To investigate this further we used this 83 bp fragment (SEQ ID No:2) and an internal 30 bp oligonucleotide (SEQ ID No:4) in gelshift experiments. Both probes were radioactively labeled and incubated with extracts from HEK293T cells and HEK293T cells transiently transfected with CMV-AP2 (Williams and Tjian, 1991). The results with the 83 bp fragment indicated that several proteins from HEK293T cell interacted with this promoter fragment. As sequence analysis revealed the presence of an AP2 motif within this 83 bp, we used extracts from AP2-transfected HEK293T cells to investigate its role. Gelshift experiments indicated that AP2 actually interacts with the 83 bp fragment and these results were confirmed by using a shorter 30 bp oligonucleotide including the AP2 site. The specificity of the interaction was demonstrated by using the cold 30 bp in a competition experiment. The use of this 30 bp oligonucleotide as competitor resulted however in a stronger binding of unknown proteins B and C. This might indicate that these proteins have an overlapping binding-motif and that binding of AP2 competes for this site. Potential candidate proteins are SREB, NRSF, Myf5, or c-ETS (Fig 5).

### Role for Barbiturates in 5-HT₇ promoter activity

According to MatInspector, a putative BARBIE (Barbiturate Inducible Element) is present in the promoter region of the 5-HT₇ receptor. To investigate the possible influence of barbiturates on 5-HT₇ promoter activity, we transiently transfected HEK293T cells with the different promoter constructs and treated the transfected cells with barbiturates for 24 h (Fig 6a). The results indicate that 24 h of barbiturate treatment (1,5 mM) repressed promoter activity in transiently transfected HEK293T cells. The reduction varied from 30 - 50 % depending on the promoter construct used and the type of barbiturate. Amobarbital seemed to be somewhat more potent and the effect was more pronounced in the 2000 promoter fragments. As barbiturates did not influence CMV promoter activity, these results indicate that the observed effect is 5-HT₇-promoter specific.
We next performed a time course experiment (Fig. 6b) which indicated that the repressive effect was already present after 6 hours and reached a maximum after 16 hours. After 52 hours promoter activity increased again.

### Role for Egr-1 and Sp1 on 5-HT₇ promoter regulation

Transient transfection of CMV-Sp1 or CMV-Egr1 together with a 5-HT7 promoter construct in NS20Y cells, showed a positive effect of both transcription factors (Egr1 and Sp1) on the 5-HT7 promoter activity (Figure 9A and 9B resepctively). In addition, the dominant negative constructs for both transcription factors (CMV-Sp1-BD and Zn-Egr1) show a negative effect on 5-HT7 promoter activity. Suggesting a role for both transcription factors in the regulation of the 5-HT7 gene.
A mutual role for both transcription factors was suggested in cotransfection experiments of CMV-Sp1 and CMV-Egr1 together with a 5-HT7 promoter construct in N2A cells. When one of the transcription factors was kept at the same level (500 ng) and the other transcription level was increased, a negative effect on the 5-HT7 promoter activity coud be seen (Figure 10A and 10B respectively).
The significance of binding by Sp1 to the 5-HT7 promoter for transcriptional activity was confirmed in experiments with mithramycin A, a compound that interferes with Sp1 binding to GC boxes in DNA. In a first experiment NS20Y cells, transiently transfected with the F1000 construct of the 5-HT7 promoter, and treated with increasing concentrations of mithramycin A, demonstrated a dose-dependent loss of promoter activity (Figure 11A). A similar phenomena was observed in 1321-N1 cells. Treating this human cell line, endogenously expressing the 5-HT7 receptor, with different concentrations of mithramycin A for 24h resulted in a significant decrease of 5-HT7 mRNA levels while the levels of GAPDH, a house-keeping gene were unaffected (Fig. 11B). These results suggest that Sp1 plays an important role in the 'in vivo' regulation of 5-HT7 promoter activity.

### DISCUSSION

We reported for the first time the full genomic structure of the human 5-HT₇ receptor including 5'- and 3'- flanking regions. The full genomic sequence including the 107 kB first intron, was cloned into an EBV-based vector, which will allow us to study the 5-HT₇ receptor gene regulation in eukaryotic cells.
By 5' RACE experiments we were able to identify a major transcriptional initiation site, which was located 307 bp upstream of the start point of translation. This site differs from a previously identified site. This could correlate with the fact that no real TATA-box of CAAT element could be identified in the promoter region so far.

Sequence analysis of the 5'-flanking region using various software programmes indicated and identified several motifs for transcription factors. Among them several putative Sp1 and AP2 sites are present which correlates with the GC-rich character of the 5'-flanking region.
We isolated promoter fragments of different lengths and tested their transcriptional activity in HEK293T cells. All fragments clearly exhibited promoter activity and our results indicated the presence of both enhancer and repressor domains. A clear enhancement of transcription was observed when a 83 bp NcoI-fragment was included in the constructs.
This 83 bp fragment was then used in gelshift experiments which indicated the presence of several interacting proteins. So far, we identified AP2 as being an enhancer region binding factor.
AP2 has been reported to play a role in promoter regulation of several other neurotransmitter receptor genes including the human m3 Muscarinic Acetylcholine receptor (Billington and Penn, 2002), the alpha7 neuronal nicotinic acetylcholine receptor (Gault et al., 1998) and the D(1A) dopamine receptor (Takeuchi et al., 1999). Competition experiments using a 30 bp fragment including the AP2 site revealed that other transcription factors could compete for overlapping sequences. These factors, for instance SREBP, NRSF, Myf5, c-ETS have their recognition sequences in the 30 bp sequence in the promoter of the 5-HT₇ receptor.
It has already been shown that the 5-HT₇ receptor is downregulated by steroids in vivo (Le Corre et al., 1997; Yau et al. 1997) and in vitro (Shimizu et al., 1997), and as this may play a role in affective disorders. The identification of the regulatory sequence elements GREF/PRE and PPAR/RXR, that are present in the 5'-flanking region, provides a tool to test the influence of steroids, for example dexamethasone , or other nuclear receptor ligands on the promoter activity and check if this difference in activity might be caused by one of the motifs. In addition, in combination with the full-length genomic clone it is now possible to study how steroids such as dexamethasone, could influence the splicing pattern of the 5-HT₇ receptor.
For depressants in general, it was known that at high doses they depress both nerve and muscle activity and inhibit oxygen consumption in the tissues. In low doses barbiturates act as sedatives, i.e. they have a tranquilizing effect; increased doses have a hypnotic or sleep-inducing effect. The mechanism of action in the central nervous system is unknown. Sequence analysis of the 5-HT₇ receptor promoter surprisingly indicated the presence of a barbiturate-inducible element (BARBIE). Our results using 1.5 mM barbiturate clearly demonstrate that barbiturates have a negative effect on 5-HT₇ promoter activity. This suggests a potential role for the 5-HT₇ receptor in sleep regulation and supports the earlier findings by Ehlen et al., 2001, who demonstrated a shift in the circadian rhythm of hamsters treated with 8-OH-DPAT, a 5-HT_{1A} agonist with high affinity for 5-HT₇ receptor. The identification of the BARBIE element now provides a tool to investigate the influence of barbitiurates on 5-HT₇ receptor regulation as well as a means to identify agents that specifically affect the barbiturate inducible element activity.
Using a SmarTest (http://www.genomatix.de) we identified a S/MAR within the 3000 bp fragment. MAR's (Matrix Attachment Regions) are believed to be responsible for the attachment of genomic DNA to the nuclear matrix or scaffold, which is a requirement to allow transcription (Bode et al., 1995). Some functional features of MARs include 1) Anchoring of regulatory elements such as promoters and enhancers to the nuclear matrix, 2) Defining the boundaries of independent chromatin domains (including all cis-regulatory elements required for coordinated expression of genes), 3) Ensuring long term activity of promoters and enhancers, 4) Insulation, rendering a functional domain insensitive to position effects. It is therefore to be expected that the full 5-HT₇ promoter regulation resides within this 3000 bp fragment.
The identification of the GC-rich region in the 5-HT7 promoter confirms earlier findings that genes that lack TATA and CAAT boxes invariable have GC-rich regions that may act in the place of the TATA box. Several of these genes have been shown to be activated by Sp1. Examples include EGF receptor [Kageyama, 1988 #38] and estrogen receptor alfa [deGraffenried, 2002 #39]. Egr-1 is being recognized and reported as both inhibiting and stimulating transcription of gene promoters at their G + C-rich elements. Sp1 is usually an activating factor but gives complex responses when in the presence of Egr-1. Competition between Sp1 and Egr-1 was first reported by Ackerman [Ackerman, 1993 #40] for the binding and activation of the ADA gene promoter. Several reports since then have indicated that there is a complex relationship between Egr-1 and Sp1. Huang showed that Egr-1 expression augments the Sp1 activation of non-overlapping Sp1 + Egr-1 sites, but inhibits Sp1 activity when the sites are overlapping by competing with Sp1 for the binding site [Huang, 1997 #41].

Our results clearly indicate a role for both transcription factors in the regulation of the 5-HT₇ receptor gene. Therefore strategies to influence the activity of these transcription factors might provide a tool to control 5-HT₇ receptor levels.
Further with this, we found that mithramycin A, a compound that interferes with Sp1 binding to GC boxes in DNA, inhibits 5-HT₇ promoter activity.

So far, only a few selective 5-HT₇ receptor antagonists have been described such as SB258719; DR4004 and SB269970 (Thomas et al., 1998; Kikuchi et al., 1999; Hagan et al., 2000), but no selective agonists are available yet. The elucidation of 5-HT₇ promoter regulation will allow us to interfere with 5-HT₇ receptor formation in a new manner and might lead to new screening strategies for drugs interfering with 5-HT₇ receptor function.
Also the fact that we have the full genomic clone available will allow us to identify the mechanism(s) responsible for the alternative splicing of the 5-HT₇ receptor. Although so far no differences between the splice variants have been reported, the possibility to interfere with the underlying mechanism might be worthwhile for future therapeutic interventions.

### References

Billington, C. K. and R. B. Penn (2002). "m3 muscarinic acetylcholine receptor regulation in the airway." Am J Respir Cell Mol Biol 26(3): 269-72.

Bode, J., T. Schlake, et al. (1995). "Scaffold/matrix-attached regions: structural properties creating transcriptionally active loci." Int Rev Cytol 162A: 389-454.

Bourson, A., V. Kapps, et al. (1997). "Correlation between 5-HT7 receptor affinity and protection against sound-induced seizures in DBA/2J mice." Naunyn Schmiedebergs Arch Pharmacol 356(6): 820-6.

Ehlen, J. C., G. H. Grossman, et al. (2001). "In vivo resetting of the hamster circadian clock by 5-HT7 receptors in the suprachiasmatic nucleus." J Neurosci 21(14): 5351-7.

Gault, J., M. Robinson, et al. (1998). "Genomic organization and partial duplication of the human alpha7 neuronal nicotinic acetylcholine receptor gene (CHRNA7)." Genomics 52(2): 173-85.

Hagan, J. J., G. W. Price, et al. (2000). "Characterization of SB-269970-A, a selective 5-HT(7) receptor antagonist." Br J Pharmacol 130(3): 539-48.

Heidmann, D. E., P. Szot, et al. (1998). "Function and distribution of three rat 5-hydroxytryptamine7 (5-HT7) receptor isoforms produced by alternative splicing." Neuropharmacology 37(12): 1621-32.

Kikuchi, C., Nagaso, H., et al. (1999). "Tetrahydrobenzindoles: selective antagonists of the 5-HT7 receptor." Journal of Medicinal Chemistry 42(4): 533-535.

Le Corre, S., T. Sharp, et al. (1997). "Increase of 5-HT7 (serotonin-7) and 5-HT1A (serotonin-1A) receptor mRNA expression in rat hippocampus after adrenalectomy." Psychopharmacology (Berl) 130(4): 368-74.

Lovenberg, T. W., B. M. Baron, et al. (1993). "A novel adenylyl cyclase-activating serotonin receptor (5-HT7) implicated in the regulation of mammalian circadian rhythms." Neuron 11(3): 449-58.

Mullins, U. L., G. Gianutsos, et al. (1999). "Effects of antidepressants on 5-HT7 receptor regulation in the rat hypothalamus." Neuropsychopharmacology 21(3): 352-67.

Saeki, Y., C. Fraefel, et al. (2001). "Improved helper virus-free packaging system for HSV amplicon vectors using an ICP27-deleted, oversized HSV-1 DNA in a bacterial artificial chromosome." Mol Ther 3(4): 591-601.

Shimizu, M., A. Nishida, et al. (1997). "Down-regulation of 5-hydroxytryptamine7 receptors by dexamethasone in rat frontocortical astrocytes." J Neurochem 68(6): 2604-9.

Sleight, A. J., C. Carolo, et al. (1995). "Identification of 5-hydroxytryptamine7 receptor binding sites in rat hypothalamus: sensitivity to chronic antidepressant treatment." Mol Pharmacol 47(1): 99-103.

Takeuchi, S., I. Imafuku, et al. (1999). "AP-2beta represses D(1A) dopamine receptor gene transcription in neuro2a cells." Brain Res Mol Brain Res 74(1-2): 208-16.

Thomas, D. R., S. A. Gittins, et al. (1998). "Functional characterisation of the human cloned 5-HT7 receptor (long form); antagonist profile of SB-258719." Br J Pharmacol 124(6): 1300-6.

Vanden Berghe, W., S. Plaisance, et al. (1998). "p38 and extracellular signal-regulated kinase mitogen-activated protein kinase pathways are required for nuclear factor-kappaB p65 transactivation mediated by tumor necrosis factor." J Biol Chem 273(6): 3285-90.

Vanhoenacker, P., G. Haegeman, et al. (2000). "5-HT7 receptors: current knowledge and future prospects." Trends Pharmacol Sci 21(2): 70-7.

Wade-Martins, R., J. Frampton, et al. (1999). "Long-term stability of large insert genomic DNA episomal shuttle vectors in human cells." Nucleic Acids Res 27(7): 1674-82.

Wade-Martins, R., R. E. White, et al. (2000). "Stable correction of a genetic deficiency in human cells by an episome carrying a 115 kb genomic transgene." Nat Biotechnol 18(12): 1311-4**.**

Williams, T. and R. Tjian (1991). "Analysis of the DNA-binding and activation properties of the human transcription factor AP-2." Genes Dev 5(4): 670-82.

Yau, J. L., J. Noble, et al. (1997). "Impact of adrenalectomy on 5-HT6 and 5-HT7 receptor gene expression in the rat hippocampus." Brain Res Mol Brain Res 45(1): 182-6.

Ying, S. W. and B. Rusak (1997). "5-HT7 receptors mediate serotonergic effects on light-sensitive suprachiasmatic nucleus neurons." Brain Res 755(2): 246-54.

## Claims

1. An isolated regulatory element of the 5-HT₇ receptor promoter region consisting of the nucleotide sequence set forth as positions -925 to -939 (SEQ ID No:3) in SEQ ID No: 1 and the complementary strand thereof;

2. A recombinant DNA molecule comprising an exogenous nucleic acid molecule according to claims 1.

3. A recombinant DNA molecule according to claim 2 wherein the isolated nucleic acid according to claim 1 is operably linked to a nucleic acid molecule that encodes a detectable product.

4. A recombinant DNA molecule according to claim 2 wherein the isolated nucleic acid molecule according to claim 1 is operably linked to a reporter plasmid containing a minimal promoter.

5. A recombinant DNA molecule according to claim 3 wherein the nucleic acid molecule that encodes a detectable product is being selected from a nucleic acid sequence encoding the 5HT₇ receptor polypeptide or a reporter gene.

6. A recombinant DNA molecule according to claim 5 wherein the reporter gene is being selected from the group consisting of the firefly luciferase gene, the β-galactosidase gene, alkaline phosphatase, the bacterial chloramphenicol acetyl transferase gene and the green fluorescent protein gene.

7. A recombinant DNA molecule according to any one of claims 2 or 5 wherein the reporter gene is the firefly luciferase gene.

8. A vector comprising the recombinant DNA molecule according to any one of claims 2 to 7.

9. A vector according to claim 8 wherein said vector is an expression vector.

10. A host cell transformed with a vector according to claims 8 or 9.

11. The host cell according to claim 10 wherein said host cell is a prokaryotic cell.

12. The host cell according to claim 10 wherein said host cell is an eukaryotic cell.

13. The host cell according to claim 10 wherein said eukaryotic cell is a mammalian cell, preferably HEK293, NS20Y or N2A cells.

14. A method for identifying compounds which are modulators of human 5HT₇ receptor promoter activity, said method comprising:
(a) contacting a host cell comprising a recombinant DNA molecule according to any one of claims 2 to 7 with a test compound; and
(b) determining whether said test compound modulates the level of expression of the detectable product.

15. A method according to claim 14 wherein the detectable product is the 5HT₇ receptor protein or a reporter gene selected from the firefly luciferase, the β-galactosidase gene, the bacterial chloramphenicol acetyl transferase gene or the green fluorescent protein gene.

16. A method for identifying compounds that modulate the activity of the barbiturate-inducible element within the 5HT₇ receptor promoter region, said method comprising:
(a) contacting a host cell transfected with a recombinant DNA molecule comprising the BARBIE element (SEQ ID No:3) operably linked to a detectable product, with a test compound, and
(b) determining whether said test compound modulates the level of expression of the detectable product.

17. A method for the identification of polypeptides which bind to nucleotide sequences involved in the biological pathway related to serotonin mediated responses, comprising the steps of:
(a) transfecting a host cell line with a recombinant DNA molecule according to any one of claims 2 to 7;
(b) transfecting the said host cell line with a variety of human cDNA sequences;
(c) identifying and isolating cells having an altered level of expression of the said reporter gene;
(d) recovering cDNA from the cells isolated in step (c); and
(e) identifying the polypeptide expressed by the cDNA recovered in step (d)
